# EUROPEAN PATENT SPECIFICATION

(11) **EP 3 950 030 B1**
(45) Date of publication and mention of the grant of the patent: **24.04.2024**
(21) Application number: 20782733.8
(22) Date of filing: 27.03.2020
(51) Int. Cl.: A61M 16/00, A61M 16/10

(54) **CONTROL SYSTEM AND METHOD FOR VENTILATION DEVICE, AND VENTILATION DEVICE**
STEUERUNGSSYSTEM UND VERFAHREN FÜR EINE BELÜFTUNGSVORRICHTUNG UND BELÜFTUNGSVORRICHTUNG
SYSTÈME ET PROCÉDÉ DE COMMANDE POUR DISPOSITIF DE VENTILATION, ET DISPOSITIF DE VENTILATION

(30) Priority: 29.03.2019 CN 201910250282
(43) Date of publication of application: 09.02.2022
(73) Proprietor: BMC Medical Co., Ltd., Shijingshan Beijing 100041 (CN)
(72) Inventor: ZHAN, Muxia, Beijing 100041 (CN); ZHANG, Anjun, Beijing 100041 (CN); ZHENG, Fang, Tianjin 301700 (CN); ZHUANG, Zhi, Beijing 100041 (CN)
(74) Representative: Grünecker Patent- und Rechtsanwälte PartG mbB
(86) International application number: PCT/CN2020/081748
(87) International publication number: WO 2020/200112

(56) References cited:
- EP-A1- 2 055 337
- EP-A2- 1 731 188
- EP-A2- 1 800 705
- WO-A1-2006/092001
- WO-A1-2010/091462
- WO-A1-2011/068418
- WO-A1-2011/077274
- WO-A1-2013/165263
- US-A1- 2008 078 387
- US-A1- 2010 094 366

## Description

### Field

The present disclosure relates to the field of medical instruments, and particularly relates to a control device and method for a ventilation therapy equipment, and ventilation therapy equipment.

### Background

Ventilation therapy equipment such as a ventilator is used as an effective means to replace, control or change the patient's physiological breathing. By increasing the lung ventilation to improve the patient's respiratory function and reduce the patient's respiration-based consumption, the ventilation therapy equipment has been commonly used for respiratory failures caused by various reasons, respiratory support treatment, anaesthetic breathing management and first-aid resuscitation, and occupies a very important position in the field of modern medicine.

According to different clinical use scenarios, the ventilation therapy equipment includes a ventilator with a pressure mode (including continuous positive pressure, automatic positive pressure, bi-level positive pressure, etc.) and a high-flow oxygen therapy instrument with a high-flow mode. Generally, a mask of the ventilator is buckled on the patient's mouth and nose to form a relatively closed space for the patient, so that air output by this equipment is supplied to the patient through the mask, and redundant air can be discharged through an exhaust hole of the mask. A tail end of a nasal oxygen tube of the high-flow oxygen therapy instrument can be plugged into the patient's nasal cavity to form an open air path for the patient. Air output from the equipment is supplied to the patient through the nasal oxygen tube, and redundant air can be discharged from a gap between the nasal oxygen tube and the nasal cavity.

The ventilation therapy equipment in the prior art has the following disadvantages: one ventilation therapy equipment usually has only one working mode, such as the pressure mode or the high flow mode, and the two working modes are realized by two different sets of ventilation therapy equipment, and cannot be switched between each other. <page 1a>

### Summary

The present disclosure aims to provide a control device and method for a ventilation therapy equipment, and ventilation therapy equipment. Corresponding working modes can be automatically switched according to different types of accessories, so that the ventilation therapy equipment is more convenient and intelligent, so that a lot of time can be saved for a doctor and a patient.

WO 2011/068418 A1 relates to breathing assistance apparatus, comprising: a gases supply unit adapted to, in use, deliver a stream of pressurised gases from an oudet, said gases supply unit further adapted to vary the pressure of said stream of pressurised gases; a supply path including a flexible self-supporting gases transportation pathway having an open first end and an open second end, said first end and said outlet adapted to connect in use so that said gases transportation pathway receives said stream of gases and conveys said gases to a patient, and a patient interface connected to said second end of said gases transportation pathway in use and adapted to receive said stream of gases and supply said patient with said gases; at least one sensor to measure at least one information parameter of a flow of gases in said supply path, and a control system associated with the gases supply unit, adapted to receive data of said at least one information parameter from said at least one sensor, said control system containing reference data, said control system comparing said received data to reference data, and determining a supply path based on said comparison.

In order to achieve the above-mentioned objective, the present invention provides a control device for a ventilation therapy equipment as defined in claim 1.

Therefore, the type of the accessory can be automatically identified according to the characteristics of the accessory itself. And thus, the corresponding working mode can be automatically switched by the recognized type of the accessory, so that a probability of mis-operation caused by manual switching is reduced, and the ventilation therapy equipment is more convenient and intelligent.

Optionally, wherein the accessory comprises a breathing tube and/or a patient interface; and the identification device is further configured for identifying the type of the accessory according to the mechanical connection characteristics of the accessory further comprises: identifying the type of the accessory, according to an electrical signal generated when a pin or an elastic sheet on a connection joint of the breathing tube is pressed down.

Optionally, wherein the accessory comprises a breathing tube and/or a patient interface; and the identification device is further configured for identifying the type of the accessory according to the electrical characteristics of the accessory further comprises: determining that the breathing tube is a non-heating tube in the case that no electrical connection signal of the breathing tube is detected, or
determining that the breathing tube is a heating tube when an electrical connection signal of the breathing tube is detected.

Optionally, wherein the accessory comprises a breathing tube and/or a patient interface; and the identification device is further configured for identifying the type of the accessory according to the air flow characteristics of the accessory further comprises: identifying the type of the accessory according to a magnitude of resistance of an air flow of the breathing tube.

Optionally, wherein the controller is further configured for determining and controlling, according to the identified type of the accessory, the ventilation therapy equipment to switch to a corresponding working state in the working mode corresponding to the type of the accessory; and the working state is related to a parameter setting in this working mode.

Therefore, the same working mode is subdivided into different working states by setting different parameter settings under the same working mode, so as to provide more targeted services to different patients, thereby improving the satisfaction of the patients.

Therefore, different parameter settings under the same working mode can be executed for different patients, and the same working mode is subdivided into different working states, so as to provide more targeted services to different patients, thereby improving the satisfaction of patients.

Correspondingly, the present invention further provides a control method for a ventilation therapy equipment as defined in claim 7.

Optionally, wherein the determining and controlling, according to the identified type of the accessory, the ventilation therapy equipment to switch to the working mode corresponding to the type of the accessory comprises: determining and controlling, according to the identified type of the accessory, the ventilation therapy equipment to switch to a corresponding working state in the working mode corresponding to the type of the accessory, wherein the working state is related to a parameter setting in this working mode.
Correspondingly, the present invention further provides a ventilation therapy equipment as defined in claim 9.

By means of the above-mentioned technical solution, the present disclosure creatively automatically switches corresponding working modes according to the identified type of the accessory of the ventilation therapy equipment, without manual switching for the corresponding working modes, so that a probability of mis-operation caused by manual switching is reduced, and the ventilation therapy equipment is more convenient and intelligent to save a lot of time for the doctor and the patient.

Correspondingly, the present invention further provides a machine readable storage medium as defined in claim 10.

Other features and advantages of the present disclosure will be described in detail in the following detailed description.

### Brief Description of Drawings

The accompanying drawings are used to provide a further understanding of the present disclosure, constitute a part of the specification, are used to explain the present disclosure together with the following specific embodiments, but do not constitute a limitation to the present disclosure. In the drawings:
FIG 1 is a structural diagram of a control device for a ventilation therapy equipment provided by an embodiment of the present disclosure;
FIG 2(a) is a structural diagram of ventilation therapy equipment in a pressure mode provided by an embodiment of the present disclosure;
FIG 2(b) is a structural diagram of ventilation therapy equipment in a flow mode provided by an embodiment of the present disclosure;
FIG 3 is a structural diagram of a connection joint between a breathing tube and a main body provided by an embodiment of the present disclosure;
FIG 4(a) is a structural diagram of electrical connection between a second type of breathing tube and a main body provided by an embodiment of the present disclosure;
FIG 4(b) is a structural diagram of electrical connection between a first type of breathing tube and a main body provided by an embodiment of the present disclosure;
FIG 5 is a flowchart of a control method for a ventilation therapy equipment provided by an embodiment of the present disclosure;
FIG 6 is a flowchart of a control method for a ventilation therapy equipment provided by an embodiment of the present disclosure; and
FIG 7 is a flowchart of a control method for a ventilation therapy equipment provided by an embodiment of the present disclosure.

Reference signs in the drawings:

| | | | |
|---|---|---|---|
| 10: | identification device | 20: | controller |
| 30: | main body component | 31: | breathing tube |
| 32: | mask | 33: | breathing tube |
| 34: | nasal oxygen tube | 35: | joint |
| 36: | joint | | |

### Detailed Description of the Embodiments

Specific embodiments of the present disclosure will be described in detail below with reference to the accompanying drawings. It should be understood that the specific embodiments described herein are only used to illustrate and explain the present disclosure, and are not intended to limit the present disclosure.

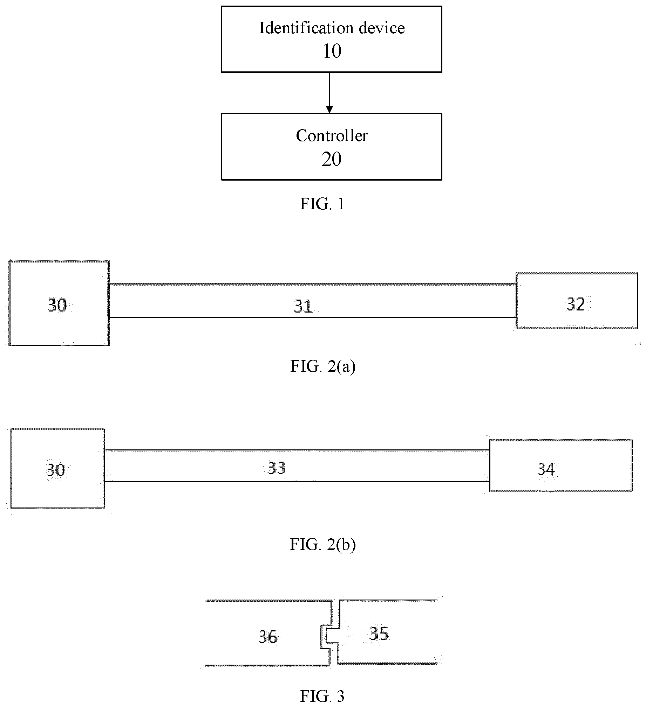

FIG 1 is a structural diagram of a control device for a ventilation therapy equipment provided by an embodiment of the present disclosure. The ventilation therapy equipment has multiple working modes. As shown in FIG. 1, the control device for a ventilation therapy equipment provided by the present disclosure includes an identification device 10 used for identifying a type of an accessory of the ventilation therapy equipment; and a controller 20 used for determining and controlling, according to the identified type of the accessory, the ventilation therapy equipment to switch to a working mode corresponding to the type of the accessory. Wherein the accessory may include a breathing tube and/or a patient interface. The type of the accessory may be divided according to different mechanical connection characteristics, electrical characteristics and/or air flow characteristics. The patient interface is a mask, an oxygen inhalation tube, a nasal oxygen tube, a nasal catheter or a tracheostomy tube.

In other embodiments, the accessory may also be a water tank or a humidifier communicating with a host of the ventilation therapy equipment.

After the accessory is mounted on the ventilation therapy equipment, the ventilation therapy equipment is turned on, and the above-mentioned control device switches the ventilation therapy equipment to be in the working mode corresponding to the type of the accessory through the identified type of the accessory. That is, after the accessory is mounted, by turning on the ventilation therapy equipment, the ventilation therapy equipment can automatically enter the working mode corresponding to the type of the accessory. In addition, in a ventilation state, after the accessory of the ventilation therapy equipment is replaced, the working mode corresponding to the type of the accessory is switched by the control device through the identified type of the accessory of the ventilation therapy equipment. That is, the control device can automatically switch the working mode, so that there is no need to manually switch the corresponding working mode, which reduces a probability of mis-operation caused by manual switching, and the ventilation therapy equipment is more convenient and intelligent, thereby saving a lot of time for a doctor and a patient. The working mode includes a pressure mode and a flow mode. Different types of accessories are provided for the two working modes respectively. For example, in the pressure mode, the main body component 30 is equipped with a first type of accessories (such as a first breathing tube 31 and a mask 32), as shown in FIG 2(a); and in the flow mode, the main body component 30 is equipped with a second type of accessories (such as a second breathing tube 33 and a nasal oxygen tube 34), as shown in FIG 2(b). The first breathing tube 31 and the second breathing circuit 33 may be the same or different. The ventilation therapy equipment in the pressure mode is equipped with the mask for treatment. An airway is kept open by providing a patient with certain inspiratory and expiratory pressures to help the patient to breathe and to improve the patient's ventilation. The ventilation therapy equipment in the flow mode is equipped with any one of the oxygen inhalation tube, the nasal oxygen tube, the nasal catheter and the tracheostomy tube for treatment. A flow output by the ventilation therapy equipment may be greater than a maximum inspiration flow of the patient to meet the patient's need for an inspiration volume.

The ventilation therapy equipment in different working modes is equipped with different types of accessories, and the identification device is also used for identifying the type of an accessory according to the mechanical connection characteristics, electrical characteristics and/or air flow characteristics of the accessory.

Next, the identification of the type of the breathing tube will be explained and described in detail in the above three aspects.

In a first case, the type of the breathing tube can be identified according to different mechanical connection characteristics of the breathing tube. As shown in FIG. 3, physical structures of connections between joints 35 of different types of breathing tubes and a joint 36 of the main body component (used for delivering air required by breath of a patient) are different. For example, connection joints of different types of breathing tubes have different pins or elastic sheets, and when the breathing tubes are connected to the main body component, different pins or elastic sheets are pressed down to generate different electrical signals. Specifically, the same kind of pins are arranged on the connection joints of the first type of breathing tubes, and an electrical signal generated is A when the pins are pressed down. The same kind of elastic sheets are arranged on the connection joints of the second type of breathing tubes, and an electrical signal generated is B when the elastic sheets are pressed down. Therefore, when the connection between the breathing tubes and the main body component are detected, if the generated electrical signal is A, it can be determined that the breathing tubes pertain to the first type, and if the generated electrical signal is B, it can be determined that the breathing tubes pertain to the second type. In this case, the identification device may include: an electrical signal receiver for receiving the generated electrical signal; and an analyzer for determining the type of breathing tube according to the received electrical signal (such as the intensity and/or the number of the electrical signal).

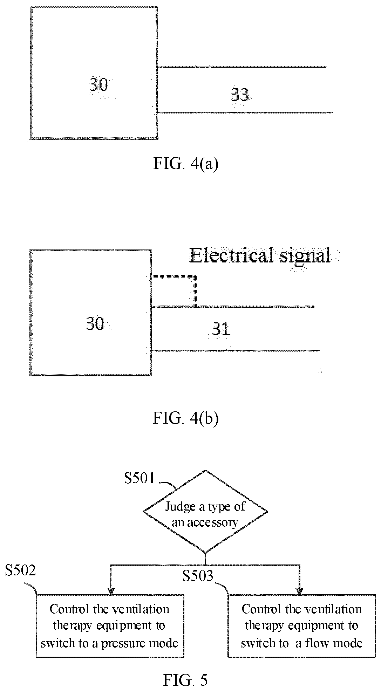

In a second case, the type of the breathing tube can be identified according to different electrical characteristics of the breathing tube. Specifically, firstly, electrical connection signals between different types of breathing tubes and the main body component are distinguished. If no electrical connection signal between the breathing tube 33 and the main body component 30 is detected, it is indicated that this type of the breathing tube 33 is a non-heating tube, and it is determined that the breathing tube is a second type of an accessory, as shown in FIG 4(a). Correspondingly, if an electrical connection signal between the breathing tube 31 and the main body component 30 is detected, it is indicated that this type of the breathing tube is a heating tube, and it is determined that the breathing tube is a first type of accessory, as shown in FIG 4(b). If an electrical connection signal between the breathing tubes and the main body component is detected for the two types of breathing tubes, it is indicated that the two types of the breathing tubes are both heating tubes, and the two types of the accessories continue to be distinguished according to specific conditions of the electrical connection signal, such as an amount of connected wires, sensor types, and internal circuit connection methods, until the two types of the breathing tubes are distinguished. In this case, the identification device may include: an electrical signal receiver for receiving an electrical signal generated by the breathing tube; and an analyzer for identifying the type according to the received electrical signal.

In a third case, the type of the breathing tube can be identified according to different air flow characteristics of the breathing tube. In the case that the two types of breathing tubes have different sizes, resistances generated by air flows passing through the breathing tubes with different sizes are also different. Specifically, sizes of the first type of breathing tubes are all the same and are A, and sizes of the second type of breathing tubes are all the same and are B (A>B). Therefore, when a resistance generated by an air flow passing through a breathing tube is detected, if the generated resistance is low, it can be determined that the breathing tube pertains to the first type of breathing tubes, and if the generated resistance is high, it can be determined that the breathing tube pertains to the second type of breathing tubes. In this case, the identification device may include: a resistance sensor for detecting the resistance of the air flow in the breathing tube; and an analyzer for identifying the type of the breathing tube based on the detected resistance of the air flow.

The above three methods for identifying the types of breathing tubes can be used alone or in combination. Of course, the present disclosure is not limited to the specific identification methods listed in the above three cases, and other specific identification methods including mechanical connection characteristics, electrical characteristics and/or air flow characteristics are also feasible. In addition, the present disclosure is not limited to identifying the type of the accessory based on the type of the breathing tube directly connected to the main body component, and can also identify the type of the accessory based on the patient interface (such as the mask and the nasal oxygen tube) indirectly connected to the main body component.

The control device further includes a display device for displaying different working modes of the ventilation therapy equipment. The display device can be a display screen which can display different technical parameters in different working modes, and can also display a fault code when the ventilation therapy equipment is in fault, which is convenient for maintenance personnel to perform maintenance.

The controller may be a general-purpose processor, a special-purpose processor, a conventional processor, a digital signal processor (DSP), multiple microprocessors, one or more microprocessors associated with a DSP core, a controller, a micro-controller, an application specific integrated circuit (ASIC), a field programmable gate array (FPGA) circuit, any other types of integrated circuits (IC), a state machine, etc.

Specifically, as shown in FIG 5, the process of controlling the ventilation therapy equipment to automatically switch to two working modes includes the following steps.

At step S501, judge a type of an accessory, in the case that the type of the accessory pertains to a first type, a step S502 is executed, and in the case that the type of the accessory pertains to a second type, a step S503 is executed.

At step S502, control the ventilation therapy equipment to switch to a pressure mode.

After the ventilation therapy equipment switched to the pressure mode, an operator can set parameters related to the pressure mode.

At step S503, control the ventilation therapy equipment to switch to a flow mode.

After the ventilation therapy equipment switch to the flow mode, an operator can set parameters related to the flow mode.

Of course, various different working modes can be set for the ventilation therapy equipment, including at least two of a continuous positive pressure mode, an automatic positive pressure mode, a bi-level positive pressure mode, a high-flow mode, a low-flow mode, etc. In the above-mentioned various working modes, the main body component is also equipped with different types of accessories, so that the types of the accessories can be identified by a similar method for identifying types of different accessories in two working modes, i.e., according to the mechanical connection characteristics, electrical characteristics and/or air flow characteristics of the accessories (related identification contents are no longer described repeatedly here). The ventilation therapy equipment is controlled, according to the identified types of the accessories, to switch to the working mode matched with the accessory. Therefore, a more comprehensive function can be realized for the switching of the ventilation therapy equipment, so that the switching is more intelligent, and the time of the operator is saved.

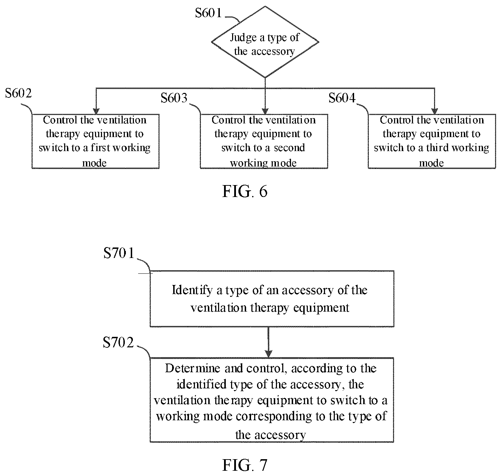

Specifically, as shown in FIG 6, the process of controlling the ventilation therapy equipment to automatically switch to multiple working modes includes the following steps.

At step S601, judge a type of an accessory, in the case that the type of the accessory pertains to a first type, a step S602 is executed. When the type of accessory pertains to a second type, a step S603 is executed. When the type of the accessory pertains to a third type, a step S604 is executed; and...
At step S602, control the ventilation therapy equipment to switch to a first working mode.
At step S603, control the ventilation therapy equipment to switch to a second working mode.
At step S604, controlled the ventilation therapy equipment to switch to a third working mode.

Of course, the first working mode, the second working mode, the third working mode and other working modes are different. For ventilation therapy equipment, the first working mode may be the continuous positive pressure mode; the second working mode may be the high-flow mode; and the third working mode may be the automatic positive pressure mode.

In another embodiment, the type of the accessory is identified by means of the identification device, and a corresponding working state in the corresponding working mode can be entered according to the type of the accessory, wherein the working state is related to a parameter setting in this working mode. Parameters include pressure, flow, pressure rising time, etc. Specifically, when the ventilation therapy equipment is used by multiple patients, the ventilation therapy equipment stores treatment modes corresponding to the multiple patients, and each treatment mode corresponds to a parameter setting of each patient. For example, a first patient corresponds to a parameter setting A in the continuous positive pressure mode; a second patient corresponds to a parameter setting B in the continuous positive pressure mode; and a third patient corresponds to a parameter setting C in the high-flow mode. When the first patient uses a matched type of an accessory, and the accessory is connected to the ventilation therapy equipment, the ventilation therapy equipment can directly enter the parameter setting A in the continuous positive pressure mode. The continuous positive pressure mode is subdivided into different parameter settings by setting different parameters such as pressure and/or pressure rising time, so that more targeted services can be provided for different patients, thereby improving the satisfaction of the patient.

According to the present invention, the accessory is also configured with a storage unit. The storage unit stores a serial number of the accessory. The controller is also used for determining and controlling, according to the type of the accessory and the serial number of the accessory, the ventilation therapy equipment to switch to the corresponding working state in the working mode corresponding to the type of the accessory, wherein the working state corresponds to the serial number. The working state is related to the parameter setting in the working mode, for example, the continuous positive pressure mode is subdivided into different working states according to the set different parameters such as the pressure and/or the pressure rising time. The serial number of the accessory can correspond to an identity (ID) label of the patient on a one-to-one basis. Preferably, the serial number of the accessory may be the ID of the patient.

Specifically, an example that a heating tube with a chip serves as the accessory is used for description. The chip of the heating tube stores a serial number (such as an ID of a patient, wherein the ID of the patient may be an ID number of the patient, the name of the patient, etc.). Before use of the ventilation therapy equipment, a correspondence relationship between the serial number (such as the ID of the patient) of the heating tube and a working state of the heating tube in a working mode corresponding to the type of the heating tube is stored in the controller in advance. When the heating tube is plugged into the ventilation therapy equipment, the controller firstly controls, according to the type of the heating tube, the ventilation therapy equipment to switch to the corresponding mode, and then controls, according to the serial number stored in the chip of the heating tube, the ventilation therapy equipment to switch to the corresponding working state in the working mode. Or, when the heating tube is plugged into the ventilation therapy equipment, the controller controls, according to the type of the heating tube and the serial number stored in the chip of the heating tube, the ventilation therapy equipment to directly switch to the corresponding working state in the corresponding working mode. Therefore, the ventilation therapy equipment can provide more targeted services to different patients, thereby improving the satisfaction of the patients. In addition, in the present disclosure, different working modes can be switched manually (by a button, a knob, or a touch screen, etc.). The significance of setting this manual switching method is that, in some occasions when no accessory is connected, if a doctor only wants to view interface information or patient's usage data in a certain working mode, the doctor can switch the equipment to be in a target mode for viewing through this manual switching mode.

In conclusion, by means of the above-mentioned technical solution, in the present disclosure, corresponding working modes can be creatively automatically switched according to the identified type of the accessory of the ventilation therapy equipment, without manual switching for the corresponding working modes, so that the probability of mis-operation caused by manual switching is reduced, and the ventilation therapy equipment is more convenient and intelligent, thereby saving a lot of time for the doctor and the patient.

Correspondingly, as shown in FIG 7, the present disclosure further provides a control method for a ventilation therapy equipment. The control method may include the following steps. At step S701, a type of an accessory of the ventilation therapy equipment is identified. At step S702, the ventilation therapy equipment is determined and controlled, according to the identified type of the accessory, to switch to a working mode corresponding to the type of the accessory.

Specific details and benefits of the control method for a ventilation therapy equipment provided by the present disclosure may refer to the descriptions of the control device for a ventilation therapy equipment, so that detailed description is omitted here.

Correspondingly, the present disclosure further provides ventilation therapy equipment. The ventilation therapy equipment may include a main body component used to deliver air required by breath of a patient; an accessory used to cooperate with the main body component to complete the delivery of the air; and the above control device for a ventilation therapy equipment.

Correspondingly, the present disclosure further provides a machine readable storage medium. The machine readable storage medium stores an instruction configured to enable a machine to execute the control method for a ventilation therapy equipment.

The machine readable storage medium includes, but is not limited to, various media that may store program codes, such as a Phase Change Random Access Memory (PRAM/RCM/PCRAM), a Static Random Access Memory (SRAM), a Dynamic Random Access Memory (DRAM), other types of RAMs, a Read Only memory (ROM), an Electrically Erasable Programmable Read-Only Memory (EEPROM), a flash memory or other internal memory technologies, a CD-ROM, a Digital Video Disk (DVD) or other optical memories, a cassette type magnetic tape, a magnetic tape/disk storage device or other magnetic storage devices.

The preferable embodiments of the present disclosure are described above in detail with reference to the accompanying drawings. However, the present disclosure is not limited to the specific details in the above embodiments. Various simple variations can be made to the technical solutions of the present disclosure within the technical concept ranges of the present disclosure, and these simple variations all fall within the protection scope of the present disclosure.

In addition, it should be noted that all the specific technical features described in the above specific embodiments can be combined in any suitable manner without conflicts. In order to avoid unnecessary repetitions, various possible combinations are not described separately in the present disclosure.

In addition, various different embodiments of the present disclosure can also be optionally combined, and these combinations should also be regarded as the content disclosed in the present disclosure, as long as they do not violate the idea of the present disclosure.

## Claims

1. A control device for a ventilation therapy equipment, the ventilation therapy equipment having multiple working modes, wherein the multiple working modes comprise a pressure mode and a flow mode and wherein the control device comprises:
an identification device (10) configured for identifying a type of an accessory (31, 32, 33, 34) of the ventilation therapy equipment; and
a controller (20) configured for determining and controlling, according to the identified type of the accessory (31, 32, 33, 34), the ventilation therapy equipment to switch to a working mode corresponding to the type of the accessory (31, 32, 33, 34),
the identification device (10) is further configured for identifying the type of the accessory (31, 32, 33, 34), according to mechanical connection characteristics, electrical characteristics and/or air flow characteristics of the accessory (31, 32, 33, 34),
wherein the accessory (31, 32, 33, 34) is configured with a storage unit which stores a serial number of the accessory (31, 32, 33, 34);
**characterised in that**
the controller (20) is further configured for determining and controlling, according to the type of the accessory (31, 32, 33, 34) and the serial number of the accessory (31, 32, 33, 34), the ventilation therapy equipment to switch to a corresponding working state in the working mode corresponding to the type of the accessory (31, 32, 33, 34), wherein the working state corresponds to the serial number, and
the working state is related to a parameter setting in this working mode.

2. The control device for a ventilation therapy equipment according to claim 1, wherein the accessory (31, 32, 33, 34) comprises a breathing tube (31, 33) and/or a patient interface (32, 34); and
the identification device (10) is further configured for identifying the type of the accessory (31, 32, 33, 34) according to the mechanical connection characteristics of the accessory (31, 32, 33, 34) further comprises: identifying the type of the accessory (31, 32, 33, 34), according to an electrical signal generated when a pin or an elastic sheet on a connection joint (35) of the breathing tube (31, 33) is pressed down.

3. The control device for a ventilation therapy equipment according to claim 1, wherein the accessory (31, 32, 33, 34) comprises a breathing tube (31, 33) and/or a patient interface (32, 34); and
the identification device (10) is further configured for identifying the type of the accessory (31, 32, 33, 34) according to the electrical characteristics of the accessory (31, 32, 33, 34) further comprises:
determining that the breathing tube (31, 33) is a non-heating tube in the case that no electrical connection signal of the breathing tube (31, 33) is detected, or
determining that the breathing tube (31, 33) is a heating tube when an electrical connection signal of the breathing tube (31, 33) is detected.

4. The control device for a ventilation therapy equipment according to claim 1, wherein the accessory (31, 32, 33, 34) comprises a breathing tube (31, 33) and/or a patient interface (32, 34); and
the identification device (10) is further configured for identifying the type of the accessory (31, 32, 33, 34) according to the air flow characteristics of the accessory (31, 32, 33, 34) further comprises: identifying the type of the accessory (31, 32, 33, 34) according to a magnitude of resistance of an air flow of the breathing tube (31, 33).

5. The control device for a ventilation therapy equipment according to any one of claims 1-4, wherein the controller (20) is further configured for determining and controlling, according to the identified type of the accessory (31, 32, 33, 34) , the ventilation therapy equipment to switch to a corresponding working state in the working mode corresponding to the type of the accessory (31, 32, 33, 34); and
the working state is related to a parameter setting in this working mode.

6. The control device for a ventilation therapy equipment according to any one of claims 1-5, wherein the multiple working modes comprise at least two of a continuous positive pressure mode, an automatic positive pressure mode, a bi-level positive pressure mode, a high-flow mode and a low-flow mode.

7. A control method for a ventilation therapy equipment, the ventilation therapy equipment having multiple working modes, wherein the multiple working modes comprise a pressure mode and a flow mode and wherein the control method comprises:
identifying (S501, S601, S701) a type of an accessory (31, 32, 33, 34) of the ventilation therapy equipment; and
determining and controlling (S502, S503, S602, S603, S604, S702), according to the identified type of the accessory (31, 32, 33, 34), the ventilation therapy equipment to switch to a working mode corresponding to the type of the accessory (31, 32, 33, 34),
the identifying the type of the accessory (31, 32, 33, 34) of the ventilation therapy equipment (S501, S601, S701) comprises
identifying the type of the accessory according to mechanical connection characteristics, electrical characteristics and/or air flow characteristics of the accessory (31, 32, 33, 34),
**characterised in that the** determining and controlling (S502, S503, S602, S603, S604, S702), according to the identified type of the accessory (31, 32, 33, 34), the ventilation therapy equipment to switch to the working mode corresponding to the type of the accessory (31, 32, 33, 34) comprises:
determining and controlling, according to the type of the accessory (31, 32, 33, 34) and a serial number configured for the accessory (31, 32, 33, 34) in a storage unit, the ventilation therapy equipment to switch to a corresponding working state in the working mode corresponding to the type of the accessory (31, 32, 33, 34), wherein the working state corresponds to the serial number, and
the working state is related to a parameter setting in this working mode.

8. The control method for a ventilation therapy equipment according to claim 7, wherein the determining and controlling (S502, S503, S602, S603, S604, S702), according to the identified type of the accessory (31, 32, 33, 34), the ventilation therapy equipment to switch to the working mode corresponding to the type of the accessory (31, 32, 33, 34) comprises:
determining and controlling (S502, S503, S602, S603, S604, S702), according to the identified type of the accessory (31, 32, 33, 34), the ventilation therapy equipment to switch to a corresponding working state in the working mode corresponding to the type of the accessory (31, 32, 33, 34),
wherein the working state is related to a parameter setting in this working mode.

9. A ventilation therapy equipment, comprising:
a main body component (30) configured for delivering air required by breath of a patient;
an accessory (31, 32, 33, 34) configured for cooperating with the main body component (30) to complete the delivery of the air; and
the control device for a ventilation therapy equipment according to any one of claims 1-6.

10. A machine readable storage medium, storing an instruction configured to enable a machine to execute the control method for a ventilation therapy equipment according to any one of claims 7-9.

## Patentansprüche

1. Steuervorrichtung für ein Beatmungstherapiegerät, wobei das Beatmungstherapiegerät mehrere Arbeitsmodi aufweist, wobei die mehreren Arbeitsmodi einen Druckmodus und einen Strömungsmodus umfassen und wobei die Steuervorrichtung umfasst:
eine Identifizierungsvorrichtung (10), die dazu konfiguriert ist, einen Typ eines Zubehörteils (31, 32, 33, 34) des Beatmungstherapiegeräts zu identifizieren; und
eine Steuerung (20), die zum Bestimmen und dazu konfiguriert ist, in Abhängigkeit von dem identifizierten Typ des Zubehörteils (31, 32, 33, 34) das Beatmungstherapiegerät so zu steuern, dass es in einen Arbeitsmodus schaltet, der dem Typ des Zubehörteils (31, 32, 33, 34) entspricht,
wobei die Identifizierungsvorrichtung (10) des Weiteren dazu konfiguriert ist, den Typ des Zubehörteils (31, 32, 33, 34) anhand von mechanischen Verbindungsmerkmalen, elektrischen Merkmalen und/oder Luftströmungseigenschaften des Zubehörteils (31, 32, 33, 34) zu identifizieren,
wobei das Zubehörteil (31, 32, 33, 34) mit einer Speichereinheit konfiguriert ist, die eine Seriennummer des Zubehörteils (31, 32, 33, 34) speichert;
**dadurch gekennzeichnet, dass**
die Steuerung (20) des Weiteren dazu konfiguriert ist, entsprechend dem Typ des Zubehörteils (31, 32, 33, 34) und der Seriennummer des Zubehörteils (31, 32, 33, 34) das Beatmungstherapiegerät so zu bestimmen und zu steuern, dass es in dem dem Typ des Zubehörteils (31, 32, 33, 34) entsprechenden Arbeitsmodus in einen entsprechenden Arbeitszustand schaltet, wobei der Arbeitszustand der Seriennummer entspricht; und
der Arbeitszustand mit einer Parametereinstellung in diesem Arbeitsmodus zusammenhängt.

2. Steuervorrichtung für ein Beatmungstherapiegerät nach Anspruch 1, wobei das Zubehörteil (31, 32, 33, 34) einen Atemschlauch (31, 33) und/oder eine Patientenschnittstelle (32, 34) umfasst; und
die Identifizierungsvorrichtung (10) des Weiteren dazu konfiguriert ist, den Typ des Zubehörteils (31, 32, 33, 34) gemäß den mechanischen Verbindungsmerkmalen des Zubehörteils (31, 32, 33, 34) zu identifizieren, was ferner Folgendes umfasst: Identifizieren des Typs des Zubehörteils (31, 32, 33, 34) gemäß einem elektrischen Signal, das erzeugt wird, wenn ein Stift oder ein elastisches Blatt an einem Verbindungsanschluss (35) des Atemschlauchs (31, 33) heruntergedrückt wird.

3. Steuervorrichtung für ein Beatmungstherapiegerät nach Anspruch 1, wobei das Zubehörteil (31, 32, 33, 34) einen Atemschlauch (31, 33) und/oder eine Patientenschnittstelle (32, 34) umfasst; und
wobei die Identifizierungsvorrichtung (10) des Weiteren dazu konfiguriert ist, den Typ des Zubehörteils (31, 32, 33, 34) anhand von elektrischen Merkmalen des Zubehörteils (31, 32, 33, 34) zu identifizieren, was ferner Folgendes umfasst:
Bestimmen, dass der Atemschlauch (31, 33) ein Nicht-Heizschlauch ist, wenn kein elektrisches Verbindungssignal des Atemschlauchs (31, 33) ermittelt wird, oder
Bestimmen, dass der Atemschlauch (31, 33) ein Heizschlauch ist, wenn ein elektrisches Verbindungssignal des Atemschlauchs (31, 33) ermittelt wird.

4. Steuervorrichtung für ein Beatmungstherapiegerät nach Anspruch 1, wobei das Zubehörteil (31, 32, 33, 34) einen Atemschlauch (31, 33) und/oder eine Patientenschnittstelle (32, 34) umfasst; und
die Identifizierungsvorrichtung (10) des Weiteren dazu konfiguriert ist, den Typ des Zubehörteils (31, 32, 33, 34) gemäß den Luftströmungseigenschaften des Zubehörteils (31, 32, 33, 34) zu identifizieren, was ferner Folgendes umfasst: Identifizieren des Typs des Zubehörteils (31, 32, 33, 34) gemäß einer Größe des Widerstands einer Luftströmung des Atemschlauchs (31, 33).

5. Steuervorrichtung für ein Beatmungstherapiegerät nach einem der Ansprüche 1 bis 4,
wobei die Steuerung (20) des Weiteren zum Bestimmen und dazu konfiguriert ist, in Abhängigkeit von dem identifizierten Typ des Zubehörteils (31, 32, 33, 34) das Beatmungstherapiegerät so zu steuern, dass es in dem dem Typ des Zubehörteils (31, 32, 33, 34) entsprechenden Arbeitsmodus in einen entsprechenden Arbeitszustand schaltet; und
der Arbeitszustand mit einer Parametereinstellung in diesem Arbeitsmodus zusammenhängt.

6. Steuervorrichtung für ein Beatmungstherapiegerät nach einem der Ansprüche 1 bis 5, wobei die mehreren Arbeitsmodi mindestens zwei aus einem kontinuierlichen Überdruckmodus, einem automatischen Überdruckmodus, einem zweistufigen Überdruckmodus, einem Hochströmungsmodus und einem Niedrigströmungsmodus umfassen.

7. Steuerungsverfahren für ein Beatmungstherapiegerät, wobei das Beatmungstherapiegerät mehrere Arbeitsmodi aufweist, wobei die mehreren Arbeitsmodi einen Druckmodus und einen Strömungsmodus umfassen und wobei das Steuerungsverfahren umfasst:
Identifizieren (S501, S601, S701) eines Typs eines Zubehörteils (31, 32, 33, 34) des Beatmungstherapiegeräts; und
Bestimmen und Steuern (S502, S503, S602, S603, S604, S702), in Abhängigkeit von dem identifizierten Typ des Zubehörteils (31, 32, 33, 34) des Beatmungstherapiegeräts so, dass es in einen Arbeitsmodus schaltet, der dem Typ des Zubehörteils (31, 32, 33, 34) entspricht,
wobei das Identifizieren des Typs des Zubehörteils (31, 32, 33, 34) des Beatmungstherapiegeräts (S501, S601, S701) umfasst:
Identifizieren des Typs des Zubehörteils anhand von mechanischen Verbindungsmerkmalen, elektrischen Merkmalen und/oder Luftströmungseigenschaften des Zubehörteils (31, 32, 33, 34),
**dadurch gekennzeichnet, dass** das Bestimmen und Steuern (S502, S503, S602, S603, S604, S702), in Abhängigkeit von dem identifizierten Typ des Zubehörteils (31, 32, 33, 34) des Beatmungstherapiegeräts so, dass es in den Arbeitsmodus schaltet, der dem Typ des Zubehörteils (31, 32, 33, 34) entspricht, umfasst:
Bestimmen und Steuern, entsprechend dem Typ des Zubehörteils (31, 32, 33, 34) und einer Seriennummer, die für das Zubehörteil (31, 32, 33, 34) in einer Speichereinheit konfiguriert ist, des Beatmungstherapiegeräts so, dass es in dem dem Typ des Zubehörteils (31, 32, 33, 34) entsprechenden Arbeitsmodus in einen entsprechenden Arbeitszustand schaltet, wobei der Arbeitszustand der Seriennummer entspricht, und
wobei der Arbeitszustand mit einer Parametereinstellung in diesem Arbeitsmodus zusammenhängt.

8. Steuerungsverfahren für einen Beatmungstherapiegerät nach Anspruch 7, wobei das Bestimmen und Steuern (S502, S503, S602, S603, S604, S702), in Abhängigkeit von dem identifizierten Typ des Zubehörteils (31, 32, 33, 34) des Beatmungstherapiegeräts so, dass es in den Arbeitsmodus schaltet, der dem Typ des Zubehörteils (31, 32, 33, 34) entspricht, umfasst:
Bestimmen und Steuern (S502, S503, S602, S603, S604, S702), in Abhängigkeit von dem identifizierten Typ des Zubehörteils (31, 32, 33, 34) des Beatmungstherapiegeräts so, dass es in dem dem Typ des Zubehörteils (31, 32, 33, 34) entsprechenden Arbeitsmodus in einen entsprechenden Arbeitszustand schaltet,
wobei der Arbeitszustand mit einer Parametereinstellung in diesem Arbeitsmodus zusammenhängt.

9. Beatmungstherapiegerät, das Folgendes umfasst:
eine Hauptkörperkomponente (30), die dazu konfiguriert ist, die von einem Patienten benötigte Atemluft abzugeben; ein Zubehörteil (31, 32, 33, 34), das dazu konfiguriert ist, mit der Hauptkörperkomponente (30) zusammenzuwirken, um die Abgabe der Luft zu vervollständigen; und
die Steuervorrichtung für ein Beatmungstherapiegerät nach einem der Ansprüche 1-6.

10. Maschinenlesbares Speichermedium, das einen Befehl speichert, der dazu konfiguriert ist, eine Maschine in die Lage zu versetzen, das Steuerungsverfahren für ein Beatmungstherapiegerät nach einem der Ansprüche 7-9 auszuführen.

## Revendications

1. Dispositif de commande pour un équipement de thérapie par ventilation, l'équipement de thérapie par ventilation ayant de multiples modes de fonctionnement, les multiples modes de fonctionnement comprenant un mode par pression et un mode par écoulement et le dispositif de commande comprenant :
un dispositif d'identification (10) conçu pour identifier un type d'un accessoire (31, 32, 33, 34) de l'équipement de thérapie par ventilation ; et
un contrôleur (20) conçu pour déterminer et commander, en fonction du type identifié d'accessoire (31, 32, 33, 34), l'équipement de thérapie par ventilation afin de basculer vers un mode de fonctionnement correspondant au type d'accessoire (31, 32, 33, 34),
le dispositif d'identification (10) étant en outre conçu pour identifier le type d'accessoire (31, 32, 33, 34), en fonction des caractéristiques de raccordement mécanique, des caractéristiques électriques et/ou des caractéristiques d'écoulement d'air de l'accessoire (31, 32, 33, 34),
l'accessoire (31, 32, 33, 34) étant conçu avec une unité de stockage qui stocke un numéro de série de l'accessoire (31, 32, 33, 34) ;
**caractérisé en ce que** le contrôleur (20) est en outre conçu pour déterminer et commander, en fonction du type d'accessoire (31, 32, 33, 34) et du numéro de série de l'accessoire (31, 32, 33, 34), l'équipement de thérapie par ventilation afin de basculer vers un état de fonctionnement correspondant dans le mode de fonctionnement correspondant au type d'accessoire (31, 32, 33, 34), l'état de fonctionnement correspondant au numéro de série ; et
l'état de fonctionnement étant lié à un réglage de paramètre dans ce mode de fonctionnement.

2. Dispositif de commande pour un équipement de thérapie par ventilation selon la revendication 1, l'accessoire (31, 32, 33, 34) comprenant un tube de respiration (31, 33) et/ou une interface patient (32, 34) ; et
le dispositif d'identification (10) étant en outre conçu pour identifier le type d'accessoire (31, 32, 33, 34) en fonction des caractéristiques de raccordement mécanique de l'accessoire (31, 32, 33, 34) comprenant en outre : l'identification du type d'accessoire (31, 32, 33, 34), en fonction d'un signal électrique généré lorsqu'une goupille ou une feuille élastique sur un joint de raccordement (35) du tube de respiration (31, 33) est pressée vers le bas.

3. Dispositif de commande pour un équipement de thérapie par ventilation selon la revendication 1, l'accessoire (31, 32, 33, 34) comprenant un tube de respiration (31, 33) et/ou une interface patient (32, 34) ; et
le dispositif d'identification (10) étant en outre conçu pour identifier le type d'accessoire (31, 32, 33, 34) en fonction des caractéristiques électriques de l'accessoire (31, 32, 33, 34) comprenant en outre :
la détermination que le tube de respiration (31, 33) est un tube non chauffant dans le cas où aucun signal de connexion électrique du tube de respiration (31, 33) n'est détecté, ou
la détermination que le tube de respiration (31, 33) est un tube chauffant lorsqu'un signal de connexion électrique du tube de respiration (31, 33) est détecté.

4. Dispositif de commande pour un équipement de thérapie par ventilation selon la revendication 1, l'accessoire (31, 32, 33, 34) comprenant un tube de respiration (31, 33) et/ou une interface patient (32, 34) ; et
le dispositif d'identification (10) étant en outre conçu pour identifier le type d'accessoire (31, 32, 33, 34) en fonction des caractéristiques d'écoulement d'air de l'accessoire (31, 32, 33, 34) comprenant en outre : l'identification du type d'accessoire (31, 32, 33, 34) en fonction d'une amplitude de résistance d'un écoulement d'air du tube de respiration (31, 33).

5. Dispositif de commande pour un équipement de thérapie par ventilation selon l'une quelconque des revendications 1 à 4, le contrôleur (20) étant en outre conçu pour déterminer et commander, en fonction du type identifié d'accessoire (31, 32, 33, 34), l'équipement de thérapie par ventilation afin de basculer vers un état de fonctionnement correspondant dans le mode de fonctionnement correspondant au type d'accessoire (31, 32, 33, 34) ; et
l'état de fonctionnement étant lié à un réglage de paramètre dans ce mode de fonctionnement.

6. Dispositif de commande pour un équipement de thérapie par ventilation selon l'une quelconque des revendications 1 à 5, les multiples modes de fonctionnement comprenant au moins deux d'un mode à pression positive continue, d'un mode à pression positive automatique, d'un mode à pression positive à double niveau, d'un mode à écoulement élevé et d'un mode à écoulement bas.

7. Procédé de commande pour un équipement de thérapie par ventilation, l'équipement de thérapie par ventilation ayant de multiples modes de fonctionnement, les multiples modes de fonctionnement comprenant un mode par pression et un mode par écoulement et le procédé de commande comprenant les étapes consistant à :
identifier (S501, S601, S701) un type d'un accessoire (31, 32, 33, 34) de l'équipement de thérapie par ventilation ; et
déterminer et commander (S502, S503, S602, S603, S604, S702), en fonction du type identifié d'accessoire (31, 32, 33, 34), l'équipement de thérapie par ventilation afin de basculer vers un mode de fonctionnement correspondant au type d'accessoire (31, 32, 33, 34),
l'identification du type d'accessoire (31, 32, 33, 34) de l'équipement de thérapie par ventilation (S501, S601, S701) consistant à
identifier le type d'accessoire en fonction des caractéristiques de raccordement mécanique, des caractéristiques électriques et/ou des caractéristiques d'écoulement d'air de l'accessoire (31, 32, 33, 34),
**caractérisé en ce que** la détermination et la commande (S502, S503, S602, S603, S604, S702), en fonction du type identifié d'accessoire (31, 32, 33, 34), de l'équipement de thérapie par ventilation à basculer vers le mode de fonctionnement correspondant au type d'accessoire (31, 32, 33, 34) consistant à :
déterminer et commander, en fonction du type d'accessoire (31, 32, 33, 34) et d'un numéro de série configuré pour l'accessoire (31, 32, 33, 34) dans une unité de stockage, l'équipement de thérapie par ventilation pour basculer vers un état de fonctionnement correspondant dans le mode de fonctionnement correspondant au type d'accessoire (31, 32, 33, 34), l'état de fonctionnement correspondant au numéro de série, et
l'état de fonctionnement étant lié à un réglage de paramètre dans ce mode de fonctionnement.

8. Procédé de commande pour un équipement de thérapie par ventilation selon la revendication 7, la détermination et la commande (S502, S503, S602, S603, S604, S702), en fonction du type identifié d'accessoire (31, 32, 33, 34), de l'équipement de thérapie par ventilation afin de basculer vers le mode de fonctionnement correspondant au type d'accessoire (31, 32, 33, 34) consistant à :
déterminer et commander (S502, S503, S602, S603, S604, S702), en fonction du type identifié d'accessoire (31, 32, 33, 34), l'équipement de thérapie par ventilation afin de basculer vers un état de fonctionnement correspondant dans le mode de fonctionnement correspondant au type d'accessoire (31, 32, 33, 34),
l'état de fonctionnement étant lié à un réglage de paramètre dans ce mode de fonctionnement.

9. Équipement de thérapie par ventilation, comprenant :
un composant de corps principal (30) conçu pour délivrer l'air requis par une respiration d'un patient ;
un accessoire (31, 32, 33, 34) configuré pour coopérer avec le composant de corps principal (30) afin d'achever la libération de l'air ; et
le dispositif de commande pour un équipement de thérapie par ventilation selon l'une quelconque des revendications 1 à 6.

10. Support de stockage lisible par une machine, stockant une instruction conçue pour permettre à une machine d'exécuter le procédé de commande pour un équipement de thérapie par ventilation selon l'une quelconque des revendications 7 à 9.
